# EUROPÄISCHE PATENTANMELDUNG

(11) **EP 0 564 782 A2**
(43) Veröffentlichungstag der Anmeldung: **13.10.1993**
(21) Anmeldenummer: 93102081.2
(22) Anmeldetag: 10.02.1993
(51) Int. Cl.: A21D 8/04, C12N 1/20, C12R 1/225

(54) **Verfahren zur Herstellung eines Weizenvorteiges**

(30) Priorität: 09.04.1992 DE 4211973
(71) Anmelder: HÜLS AKTIENGESELLSCHAFT, D-45764 Marl (DE)
(72) Erfinder: Hill, Frank, Dr., W-4020 Mettmann (DE)

(57) **Zusammenfassung**

Zur Herstellung eines pumpfähigen und lagerstabilen Weizenvorteiges mit hohem Zucker- und geringem Säuregehalt wird in Wasser suspendiertes, thermisch aufgeschlossenes Weizenmehl mit Bakterien der Gruppen Streptococcaceae oder Lactobacillaceae fermentiert.

## Beschreibung

Die Erfindung betrifft ein neues Verfahren zur Herstellung eines lagerstabilen und pumpfähigen Weizenvorteiges mit hohem Mono- und Disaccharidgehalt sowie die Verwendung dieses Vorteiges für Weizenbackwaren.

Weizenvorteige werden im allgemeinen zur Herstellung von qualitativ hochwertigen Backwaren eingesetzt.

Backwaren, die aus Roggenmehl hergestellt werden, benötigen meist eine mehrstufige Führung des Teiges und eine Säurebehandlung. Bei einer zweistufigen Führung wird dabei zunächst ein Vorteig gesäuert, der dann dem Brotteig zugemischt wird.

In DE-C-26 11 972 wird auch eine einstufige Teigführung zur Herstellung von Roggenschrotbrot beschrieben. Dabei wird ein flüssiger Sauerteig eingesetzt, der bis zur Einstellung der bakteriologischen Stoffwechselaktivität der Sauerteig-Bakterien ausgesäuert ist und dabei einen hohen Säuregrad aufweist.

Backwaren, die aus Weizenmehl hergestellt werden, benötigen dagegen keine mehrstufige Führung des Teiges und auch keine Säurebehandlung. Die meisten Weizenbackwaren werden deshalb in einstufiger Teigführung hergestellt. Dabei kann die Qualität der Weizenbackwaren durch den Zusatz handelsüblicher Backmittel verbessert werden.

Teilweise werden die Backmittel auch in das Weizenmehl eingemischt und als sogenannte "Fertigmehle" vertrieben. Die Verwendung von Weizenbackmitteln und Fertigmehlen ergibt zwar qualitativ verbesserte Weizenbackwaren, verursacht aber höhere Kosten und führt zu einem uniformen Produkt mit dem sich handwerklich geführte Herstellbetriebe nicht mehr von industriellen Herstellern absetzen können.

Es ist außerdem bekannt, daß Vorteigführungen auch ohne die Verwendung von Weizenbackmitteln qualitativ verbesserte Weizenbackwaren ergeben können. Die bekannten Vorteigführungen, wie Quellstück, Brühstück oder der amerikanische "liquid sponge", sind zeitlich kurze, nicht lagerstabile Vorteige. Dagegen sind Weizensauerteige, wie z. B. das spanische "masa madre" und der amerikanische "San Francisco sour dough", zeitlich lange geführte, lagerstabile Vorteige. Die zuletzt genannten Weizenvorteige haben aber einen hohen Säuregrad, der im fertigen Gebäck eine nicht immer gewünschte saure Geschmacksrichtung verleiht.

Durch Zusatz von Amyloglucosidase und α-Amylase kann gemäß Die Nahrung 28 (8), 837 - 49, (1984), der Gehalt an reduzierenden Zuckern in Sauerteigen erhöht werden. Derartige Sauerteige sind aber bisher wegen des hohen Zukkergehaltes wenig lagerstabil.

In Getreide, Mehl und Brot 41 (4), 118 - 22, (1987), werden die mikroorganismen von Spontansauern untersucht. Danach besteht die Mikroflora von reifen und übersäuerten Spontansauerteigen überwiegend aus Pediococcus acidilactici. Derartige Sauerteige führen jedoch wegen eines geringen Zuckergehaltes und eines hohen Säuregrades in Weizenbackwaren zu einem sauren Geschmack.

Nach Getreide, Mehl und Brot 38 (7), 203 - 205, (1984), werden aber auch mehrstufige Teigführungen angewandt, um bestimmte Eigenschaften von Heizenbackwaren zu verbessern. Die mehrstufigen Teigführungen sind jedoch umständlich und erfordern einen erheblichen Mehraufwand an Zeit.

Wegen des hohen Arbeitsaufwandes, der geringen Lagerfähigkeit oder wegen der hohen Säuregrade sind diese Weizenvorteige zur Herstellung hochwertiger Backwaren in handwerklich geführten Bäckereien nur schwierig einsetzbar.

Zur Herstellung von Weizensauerteigen werden nach Getreide, Mehl und Brot 43 (2), 49 - 52, (1989), Starterkulturen aus 2 oder 3 Lactobacillus-Stämmen vorgeschlagen. Danach sind auch eine Woche lager- und gebrauchsfähige flüssige Sauerteige herstellbar. Diese Sauerteige sind jedoch nur wegen eines niedrigen Anteils an vergärbaren Kohlenhydraten lagerfähig. Außerdem werden nur sehr geringe Säuregrade von 3,5 bis 4 eingestellt, um den Weizenbrotgeschmack nicht zu beeinträchtigen. Für Weizensauerteige mit hohem Zuckergehalt gibt diese Schrift keine Anregungen.

Aufgabe der vorliegenden Erfindung war es daher, einen einfach handhabbaren, zuckerreichen Weizenvorteig lagerfähig zu machen, ohne eine starke Säuerung, alkoholische Gärung oder anderweitigen mikrobiellen Verderb aufkommen zu lassen.

Die Aufgabe wird erfindungsgemäß durch die Maßnahmen von Anspruch 1 gelöst.

Überraschend wurde gefunden, daß Bakterien der Gruppen Streptococcaceae und Lactobacillaceae den pH-Wert von zu 10 bis 100 % thermisch aufgeschlossenem Mehl in Wasser innerhalb von 1 bis 2 Tagen bei Raumtemperatur auf ca. 4 abzusenken vermögen, dabei aber nur eine geringe Menge Säure bilden. Die Weizenvorteige weisen deshalb auch nach fünftägiger Fermentation bei Raumtemperatur nur niedrige Säuregrade auf. Die erfindungsgemäßen Weizenvorteige weisen Säuregrade von vorzugsweise 5 bis 8 auf.

Bevorzugt werden Bakterien des Lactobacillaceae-Stammes Pediococcus DSM 6869 verwendet.

In einer bevorzugten Ausführungsform werden dabei trockene und lagerstabile Reinkulturpräparate dieser Bakterien eingesetzt. Die Trocknung der Präparate kann hier nach der Lehre von DE 33 24 644 durchgeführt werden.

Mit Pediococcus DSM 6869 wird auch nach fünftägiger Fermentation bei Raumtemperatur ein Säuregrad von 7 nicht überschritten.

Der Mono- und Disaccharidgehalt des Weizenvorteiges besteht überwiegend aus Glucose und Maltose. Andere Mono- und Disaccharide, wie z. B. Fructose, Mannose oder Saccharose, können in geringeren Mengen ebenfalls enthalten sein. Der Maltosegehalt beträgt vorzugsweise 20 bis 120 g pro kg Weizenvorteig.

Zur Herstellung von zu 10 bis 100 % thermisch aufgeschlossenem Weizenmehl wird das Mehl in wäßriger Suspension auf mindestens 60 °C erhitzt. Dabei wird die Stärke teilweise oder vollständig verkleistert. Unter Anwendung von Druck kann das Mehl auch bei Temperaturen bis etwa 150 °C aufgeschlossen werden. Da Stärken, abhängig von ihrem Amylopektingehalt, Verkleisterungstemperaturen von meist 60 bis 65 °C aufweisen, werden vorzugsweise Temperaturen von 65 bis 100 °C angewandt, wobei im allgemeinen 15 Sekunden bis 10 Minuten benötigt werden.

Zu 10 % thermisch aufgeschlossenes Weizenmehl liegt vor, wenn 10 % der Stärke verkleistert ist. Bei 100 % thermische aufgeschlossenem Weizenmehl ist die gesamte Stärke verkleistert. Ein thermisch aufgeschlossenes Weizenmehl ist Paniermehl.

Für das erfindungegemäße Verfahren kann man aufgeschlossenes Weizenmehl in Wasser suspendieren. Man kann aber auch Weizenmehl zunächst in Wasser aufschließen und die dabei erhaltene Suspension direkt einsetzen.

Durch die diastatischen Enzyme des Mehls setzt in Wasser die Verzuckerung der verkleisterten Stärke ein, die bei Raumtemperatur in 1 bis 2 Tagen beendet ist. Die Verzuckerung der unverkleisterten Stärke läuft erheblich langsamer ab.

Vorzugsweise werden vor der Fermentation zu der wäßrigen Suspension, die 10 bis 70 Teile Weizenmehl, das zu 10 bis 100 % thermisch aufgeschlossen ist, enthält, weitere 90 bis 30 Teile thermisch nicht vorbehandeltes Weizenmehl bei Temperaturen zwischen 10 und 60 °C eingerührt. Wird dabei ein Vorteig mit geringer Süßkraft gewünscht, so verwendet man eine Suspension mit überwiegend thermisch nicht vorbehandeltem Weizenmehl. Soll dagegen ein süßer Vorteig hergestellt werden, so setzt man eine Suspension mit überwiegend aufgeschlossenem Weizenmehl an.

Zusätzlich können dem Ansatz noch Enzyme, wie Amyloglucosidase, zugefügt werden, die die bei der Verzuckerung gebildete Maltose zu Glucose abbauen und dadurch die Süßkraft weiter verstärken. Wenn Amyloglucosidase verwendet wird, so wird dieses Enzym in Mengen von 0,001 bis 1 %, bezogen auf die insgesamt eingesetzte Mehlmenge, zugemischt.

Dem Weizenvorteig können auch, bezogen auf das insgesamt eingesetzte Weizenmehl, bis zu 25 % übliche backwirksame Stoffe zugesetzt werden. Geeignet sind beispielsweise
0,001 bis 1 % Enzyme, wie Xylanase,
0,1 bis 10 % Hydrokolloide, wie Guarmehl,
0,1 bis 10 % Emulgatoren, wie Lecithin,
0,5 bis 5 % Proteine, wie z. B. Weizenkleber, oder
1 bis 10 % Kochsalz.

Dabei können auch Mischungen dieser Komponenten eingesetzt werden. Die Gesamtmenge liegt, wenn diese Stoffe zugesetzt werden, vorzugsweise bei 1 bis 20 %.

Diese backwirksamen Stoffe können dem Vorteig direkt zugesetzt werden. In einer bevorzugten Ausführungsform werden sie aber als trockene und rieselfähige Abmischung mit dem Bakterienpräparat (Starterkulturpräparat) dem verzuckerten Weizenmehl zugefügt.

Das erfindungsgemäße Verfahren liefert einen Vorteig, der bei nur geringen Veränderungen über mehr als 5 Tage bei Raumtemperatur gelagert werden kann, der bei einer Teigausbeute von > 200 gut rühr- und pumpfähig ist und dadurch leicht dosiert werden kann.

Die während der Fermentation gebildete L-Milchsäure und Ethanol verleihen dem Vorteig zusammen mit dem gebildeten Zucker einen milden süß-sauren Geschmack. Eine unerwünschte starke Säuerung wird vermieden. Darüber hinaus bietet das vorliegende Verfahren die Möglichkeit, durch geringe Änderungen in der Betriebsweise ein breites Spektrum von Qualitätsmerkmalen bei Weizenbackwaren zu beeinflussen.

Der Vorteig wird zur Herstellung von Weizenbackwaren verwendet. Dabei setzt man vorzugsweise so viel Vorteig dem Teig zu, daß 10 bis 25 % der gesamten Mehlmenge des dann gebildeten Teiges aus dem Vorteig stammen. Dieser Zusatz verbessert in den fertigen Backwaren das Aroma, die Frischhaltung und die Bräunung. Zu hohe Vorteigmengen beeinträchtigen jedoch das Volumen der Weizenbackwaren.

Weizenbackwaren im Sinne der Erfindung sind Backwaren, die überwiegend mit Weizenmehl hergestellt wurden. Beispiele dafür sind Weißbrote, Toastbrote, Brötchen und Pizzas. Vorzugsweise wird die Erfindung jedoch auf Weißbrote und Brötchen angewandt.

Vorzugsweise wird bei der Herstellung des Weizenvorteiges so verfahren, daß zunächst heißes Wasser in einem Behälter mit Rührwerk vorgelegt wird. Dann wird ein Teil des Mehls bei Temperaturen > 65 °C eingerührt. Nach dem Absinken der Temperatur unter den Verkleisterungspunkt wird die restliche Mehlmenge zugesetzt. Danach wird ein Bakterienpräparat eingemischt, worauf mehrere Tage lang bei Raumtemperatur leicht gerührt wird.

Die folgenden Beispiele sollen die Erfindung verdeutlichen.

### Beispiel 1

### Isolierung des Bakteriums Pediococcus DSM 6869

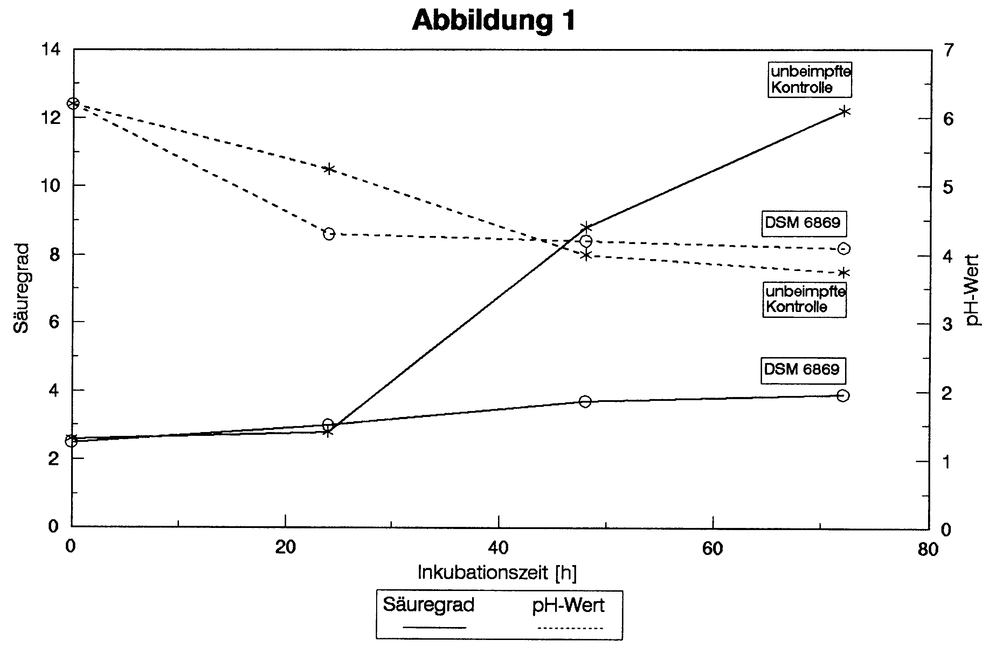

Dieser Stamm wird mit einer Reihe anderer Lactobazillen aus Silageproben (eingefroren bei -18 °C) nach folgendem Vorgehen isoliert: 
10 mg Silage + 1,5 ml Wasser + 1 g Weizenmehl
24 h bei 30 °C, anaerob
↓
Ausstrich auf Nähragar
(10 % Mehl + 2 % Agar)
72 h bei 30 °C, anaerob
↓
Reinigungsausstrich auf Indikatoragar für Säurebildung
(Zusammensetzung des Agars: 1 % Hefeextrakt, 1 % Sojapepton, 2 %
Glucose, 2 % Agar. Der pH-Wert wird auf 6,5 eingestellt. Die flüs-
 
sige Agarlösung wird in zwei Schichten in Petrischalen ausgegossen.
Die obere Schicht enthält zusätzlich 0,5 % CaCO3.)
48 h bei 30 °C, semi-aerob
Die so erhaltenen Milchsäurebakterien werden in flüssigen Mehlteigen auf rasche aber geringe Säurebildung geprüft. Der isolierte Stamm Pedicococcus DSM 6869 erreicht nach 24 Stunden Bebrütung im flüssigen Vorteig einen pH-Wert Von 4,3 und säuert bei längerer Bebrütung kaum unter pH 4,0. Die unbeimpfte Kontrolle säuert in 24 Stunden kaum, danach setzt kräftige Säuerung ein, die nach 72 Stunden Bebrütung wesentlich stärker ist als die Säuerung von Pediococcus DSM 6869. Der Säuregrad liegt bei Pediococcus DSM 6869 nach 72 Stunden bei 4, während die unbeimpfte Kontrolle dann einen Säuregrad von über 12 aufweist (vgl. Abbildung 1).

Zur Herstellung einer trockenen und lagerstabilen Starterkultur wird DSM 6869 in einem flüssigen Nährmedium der folgenden Zusammensetzung vermehrt: 5 % Maltosesirup, 1 % Hefeextrakt, 1 % Pepton und 0,5 % Calciumcarbonat. Die Nährlösung wird nach dem Beimpfen gerührt und mit 0,2 vvm schwach belüftet. Es wird bei 30 °C inkubiert. Die Zellsuspension wird im Wirbelbetttrockner auf Maltodextrin aufgesprüht. Die Temperatur der entfeuchteten Zuluft beträgt 45 °C. Das Starterkulturpräparat wird auf eine Trockensubstanz von 95 % getrocknet. Die Anzahl der lebensfähigen Bakterien in dem trockenen und rieselfähigen Präparat beträgt 1 x 10¹⁰ KbE/g (KbE: Kolonie bildenden Einheiten).

### Beispiel 2

In einen 10-l-Behälter werden 3,8 l Wasser von 72 °C gegeben. Darin wird unter Rühren mit einem Ankerrührer 1 kg Weizenmehl Typ 550 verteilt. Nachdem die viskose Suspension auf 40 °C abgekühlt ist, wird ein weiteres kg Weizenmehl Typ 550 eingerührt. Dann werden 10 g Starterkultur des Stammes Pediococcus DSM 6869 mit 1 x 10¹⁰ KbE/g zugegeben. Es wird unter Rühren für 5 Tage bei Raumtemperatur inkubiert. Täglich werden Säuregrad, pH-Wert und der Maltosegehalt im Ansatz bestimmt. Die Bestimmung des Säuregrades erfolgt in 10 g flüssigem Vorteig durch Titration mit 0,1 N NaOH (Spicher und Stephan in Handbuch Sauerteig - Biologie, Biochemie, Technologie, Seite 316 ff., 1982). Angegeben als Säuregrad sind die verbrauchten ml 0,1 N NaOH, die zum Einstellen von pH 8,5 notwendig sind.

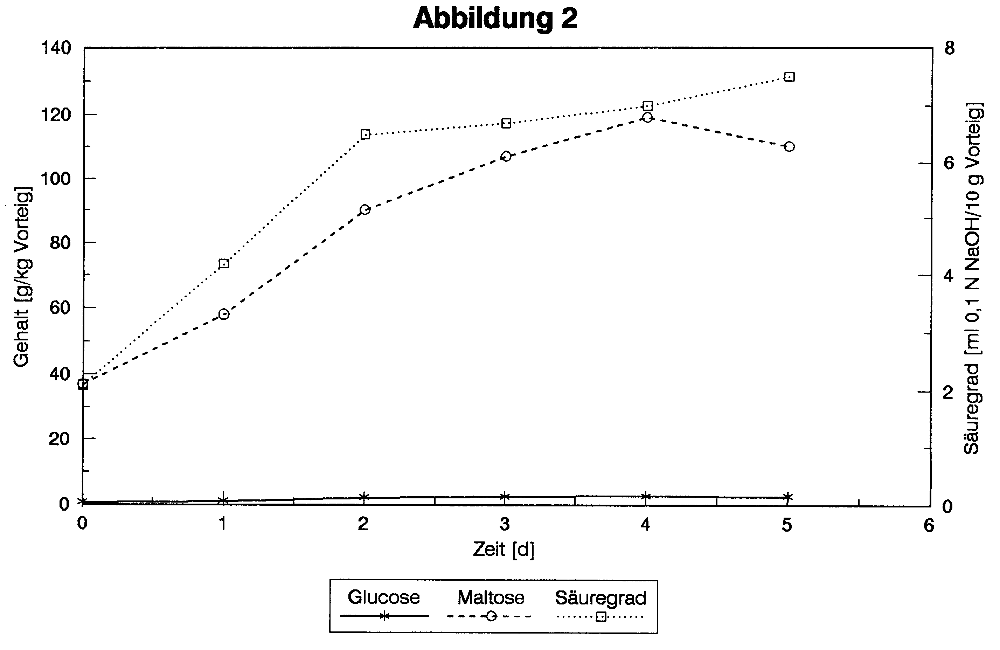

Nach 2 Tagen ist ein relativ stabiler, maltosereicher Vorteig entstanden. Der Säuregrad von 6,5, der pH-Wert von 4,0 und der Maltosegehalt von 90 g/kg Vorteig ändern sich bei weiterer Lagerung nur wenig (vgl. Abbildung 2). Der Gehalt an Milchsäure und Ethanol beträgt nach 2 Tagen jeweils ca. 1 g/kg Vorteig.

Mit einem Weizenvorteig, der wie oben beschrieben bereitet ist, werden nach 3 Tagen Testbrötchen gebacken, die mit Brötchen, die ohne Weizenvorteig hergestellt wurden, nachfolgend verglichen werden.

Die Rezepturen:

| Zutaten | Ansatz 1 (Kontrolle) direkte Führung ohne Zusatz (g) | Ansatz 2 2stufige Führung mit maltosereichem Vorteig (g) | Ansatz 3 (Kontrolle) direkte Führung mit Backmittel (g) |
|---|---|---|---|
| Weizenmehl Typ 550 | 86 | 69 | 86 |
| Backmittel* | - | - | 2,5 |
| Salz | 1,2 | 1,2 | 1,2 |
| Weizenvorteig | - | 50 | - |
| Wasser | 48 | 18 | 50 |
| Preßhefe | 1 | 1 | 1 |

| | | | |
|---|---|---|---|
| * handelsübliches Weizenbackmittel | | | |

Die Zutaten werden zu einem Teig verknetet und ausgeformt, dann 30 Minuten bei 30 °C auf Gare gestellt und anschließend bei 220 °C gebacken (25 Minuten). Die ausgebackenen Brötchen werden wie folgt bewertet:

| Beurteilung | Ansatz 1 | Ansatz 2 | Ansatz 3 |
|---|---|---|---|
| Volumen | + | + | ++ |
| Bräunung | + | ++ | ++ |
| Geschmack | + | ++ | ++ |
| Frischhaltung* | + | ++ | ++ |

| | | | |
|---|---|---|---|
| * Weichheit der Krume nach 24 Stunden Lagerung | | | |
| Dabei bedeutet hier und in den folgenden Beispielen + zufriedenstellend ++ gut +++ sehr gut | | | |

Das Ergebnis zeigt, daß mit der Verwendung eines maltosereichen Weizenvorteiges bei der Herstellung von Weizengebäck mit Ausnahme des Gebäckvolumens die Gebäckeigenschaften einer unter Zusatz eines handelsüblichen Weizenbackmittels hergestellten Backware erreicht werden.

### Beispiel 3

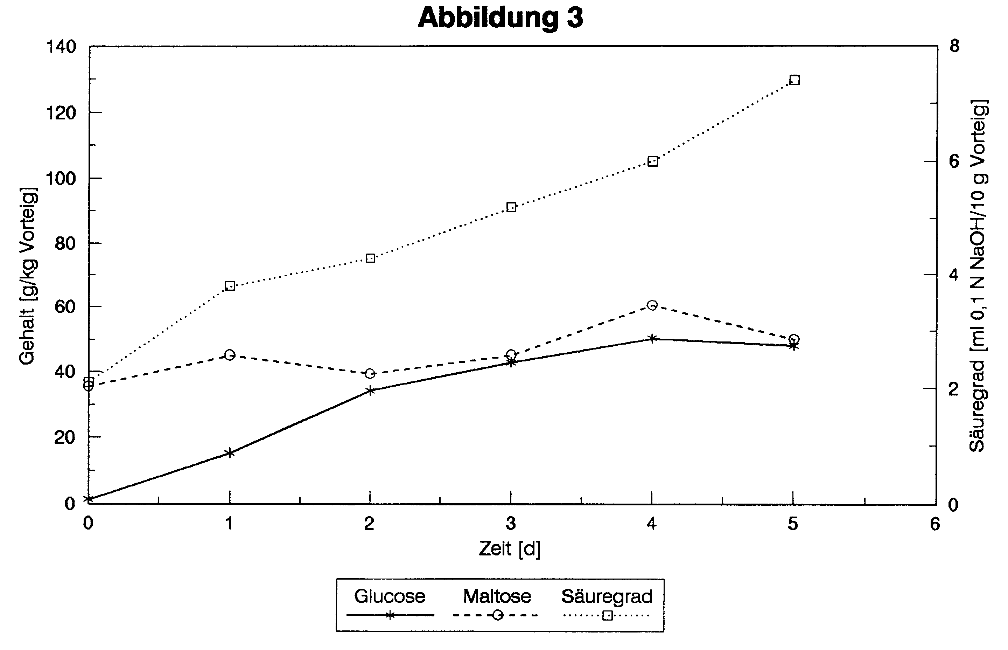

Es wird ein Weizenvorteig wie in Beispiel 2 angegeben bereitet, mit der Abänderung, daß mit der Starterkultur 300 mg eines handelsüblichen Präparates von Amyloglucosidase mit einer Aktivität von 1 200 U/g dem Vorteig zugefügt werden. Nach 3 Tagen Inkubation werden ein pH-Wert von 4,2, ein Säuregrad von 5,2, ein Maltosegehalt von 45 g/kg und ein Glucosegehalt von 43 g/kg bestimmt (vgl. Abbildung 3). Die mit diesem Vorteig hergestellten Brötchen werden wie folgt beurteilt:

| | |
|---|---|
| Volumen: | ++ |
| Bräunung: | +++ |
| Geschmack: | +++ |
| Frischhaltung: | ++ |

### Beispiel 4

Es wird ein Weizenvorteig wie in Beispiel 3 angegeben bereitet, mit der Abänderung, daß außer Starterkultur und Amyloglucosidase 4 g pulvriges Sojalecithin der Fa. Lukas Meyer, D-2000 Hamburg, zugesetzt werden. Nach 3 Tagen Inkubation wird ein Säuregrad von 6,8, ein pH-Wert von 3,9, ein Maltosegehalt von 72 g/kg und ein Glucosegehalt von 39 g/kg bestimmt. Die mit diesem Vorteig gebackenen Brötchen werden wie folgt beurteilt:

| | |
|---|---|
| Volumen: | +++ |
| Bräunung: | +++ |
| Geschmack: | +++ |
| Frischhaltung: | ++ |

### Beispiel 5

Es wird ein Weizenvorteig wie in Beispiel 4 beschrieben angesetzt, mit der Abänderung, daß außer Starterkultur, Amyloglucosidase und Lecithin 2 g des Hydrokolloids Xanthan zugesetzt werden. Nach 3 Tagen wird ein Säuregrad von 6,3, ein pH-Wert von 4, ein Maltosegehalt von 66 g/kg sowie ein Glucosegehalt von 36 g/kg bestimmt. Die mit diesem Vorteig gebackenen Brötchen werden wie folgt beurteilt:

| | |
|---|---|
| Volumen: | +++ |
| Bräunung: | +++ |
| Geschmack: | +++ |
| Frischhaltung: | +++ |

## Patentansprüche

1. Verfahren zur Herstellung eines pumpfähigen und lagerstabilen Weizenvorteiges mit einem Mono- und Disaccharidgehalt von 30 bis 200 g/kg Weizenvorteig,
dadurch gekennzeichnet,
daß man in Wasser suspendiertes, zu 10 bis 100 % thermisch aufgeschlossenes Weizenmehl mit Bakterien der Gruppen Streptococcaceae und/oder Lactobacillaceae bis zu einem Säuregrad von 5 bis 10 fermentiert.

2. Verfahren nach Anspruch 1,
dadurch gekennzeichnet,
daß man mit dem Lactobacillaceae-Stamm Pediococcus DSM 6869 fermentiert.

3. Verfahren nach Anspruch 1,
dadurch gekennzeichnet,
daß der Gehalt des Disaccharids Maltose bei 20 bis 120 g/kg Weizenvorteig liegt.

4. Verfahren nach Anspruch 1,
dadurch gekennzeichnet,
daß dem aufgeschlossenen Weizenmehl zusätzlich Amyloglucosidase zugegeben wird.

5. Verfahren nach Anspruch 1,
dadurch gekennzeichnet,
daß man zur Fermentation neben 10 bis 70 Teilen aufgeschlossenem Weizenmehl 90 bis 30 Teile thermisch nicht vorbehandeltes Weizenmehl bei einer Temperatur zwischen 10 und 60 °C in Wasser suspendiert.

6. Verfahren nach Anspruch 1,
dadurch gekennzeichnet,
daß man bis zu einem Säuregrad von 5 bis 8 fermentiert.

7. Verfahren nach den Ansprüchen 1 und 5,
dadurch gekennzeichnet,
daß man zusätzlich, bezogen auf das insgesamt eingesetzte Weizenmehl, bis zu 25 % übliche backwirksame Stoffe zugibt.

8. Verwendung des Weizenvorteiges nach Anspruch 1 zur Herstellung von Weizenbackwaren.

9. Bakterien des Stammes Pediococcus DSM 6869.
